# EUROPEAN PATENT SPECIFICATION

(11) **EP 2 950 860 B1**
(45) Date of publication and mention of the grant of the patent: **03.10.2018**
(21) Application number: 14773911.4
(22) Date of filing: 03.03.2014
(51) Int. Cl.: A61M 11/02, A61M 11/06, A61M 15/02, A61M 15/00

(54) **NEBULIZER FOR INFANTS AND RESPIRATORY COMPROMISED PATIENTS**
ZERSTÄUBER FÜR KINDER UND ATMUNGSBEEINTRÄCHTIGTE PATIENTEN
NÉBULISEUR POUR NOURRISSONS ET PATIENTS ATTEINTS DE PROBLÈMES RESPIRATOIRES

(30) Priority: 14.03.2013 US 201313830498
(43) Date of publication of application: 09.12.2015
(73) Proprietor: MicroDose Therapeutx, Inc., Ewing, NJ 08628 (US)
(72) Inventor: GUMASTE, Anand, West Windsor, NJ 08550 (US); FLEMING, Scott, Ewing, NJ 08628 (US); CHAN, Philip, Hightstown, NJ 08520 (US)
(74) Representative: Anderson, James Edward George
(86) International application number: PCT/US2014/019965
(87) International publication number: WO 2014/158764

(56) References cited:
- CA-A1- 2 811 947
- US-A- 6 026 809
- US-A- 6 120 441
- US-A1- 2004 050 860
- US-A1- 2004 123 864
- US-A1- 2009 090 361
- US-A1- 2009 165 788
- US-A1- 2009 165 790
- US-A1- 2011 000 481
- US-A1- 2011 000 481
- US-A1- 2012 255 548
- US-B2- 7 793 660

## Description

This invention relates to a device for and dry nebulization of an aerosolizable material. The invention has particular application to delivery of powdered pharmaceutical preparations to infants and respiratory compromised patients and will be described in connection with such utility, although other utilities are contemplated.

A majority of the drugs used to treat asthma and chronic obstructive pulmonary disease (COPD) are inhaled. Recently, however, there has been a move to deliver drugs to the lungs to treat other diseases, such as diabetes, through systemic absorption. The delivery of the drug to the lungs requires that the drug be in the form of a fine aerosol suitable for inhalation. It is the opinion of the pharmaceutical industry that the particles in the aerosol need be between 1 to 5 microns in size for effective delivery and absorption. These particles in the aerosol may be either in a dry powder format or droplets of a liquid medium having the drug suspended or dissolved in it. The general advantages of pulmonary delivery are avoidance of first pass metabolism, site specific delivery of the drug, potential higher bio availability, etc. Three types of devices have been traditionally used to create the aerosol needed for pulmonary delivery- metered dose inhalers (MDIs), dry powder inhalers (DPIs) and aqueous nebulizers.

MDIs have a pressurized canister filled with a liquid propellant. The drug is either suspended or dissolved in the propellant. The MDIs have a metering valve for metering out a known quantity of the propellant and hence the drug. When the canister is depressed against the MDI housing a known quantity of the propellant is discharged. The propellant evaporates leaving behind a fine aerosol of the drug suitable for inhalation by the patient. For effective delivery of the drug to the lungs the patient needs to co-ordinate breath inhalation with the discharge of the drug from the canister. Patients are not always effective in achieving this co-ordination leading to dose variability. Incorporation of a breath actuation mechanism addresses this concern but the variability still exists because of the "cold" freon effect where the patient stops breathing when the cold aerosol hits the back of the throat. This is especially true of the pediatric patients where co-ordination is of major concern. To overcome these limitations and to minimize the variability of the dose delivered, the MDI is normally recommended to be used with a spacer especially for children. This primary function of the spacer is to slow down the MDI discharge and function as a holding chamber for the aerosol plume. A face mask may be attached to the end of the spacer. These spacers normally are made of plastic and therefore tend to build up electrostatic charge on the inside surface of the spacer. The large dead space between the inlet and outlet of the spacer coupled with the electrostatic charge has the effect of lowering the amount of dose delivered and the amount of drug that is in the respirable range. It is estimated that MDIs deliver about 10% to 20% of the dose to lungs in adults with good co-ordination. Studies have shown that for pediatric patients between 3 years to 5 years using an MDI with a spacer and face mask, the lung delivery is <10% of the dose.

In DPIs the drug is micronized to the right size required for pulmonary delivery. If the drug is potent it normally is mixed with an excepient such as lactose. When drugs are micronized to this size they tend to aggregate. As mentioned above, it is commonly accepted in the pharmaceutical industry that particle sizes, as a unit or in aggregate, need to be between 1 and 5 micron for effective delivery to the lungs. The aggregates are dispersed into an aerosol by introducing the drug into a strong airflow. The airflow needed to disperse the powder typically is high ranging from 30 L/min to 90 L/min. Failure to establish this airflow can result in a lower dose being delivered to the lungs. Any inconsistency in the breathing will lead to variability in dose delivered. As an example a so-called Turbuhaler inspiratory flow-driven inhaler has been developed and is approved for children 6 years and above delivers 20- 30 % of the drug to the lungs when the airflow established by the patient is 60 L/min. However when the airflow drops to 36 L/min the amount of drug delivered is only 15%. The patient must therefore use rapid deep inhalation to adequately disperse the powder. This may not be possible for infants, young children and respiratory compromised patients of any age. Besides the inability of these patients to establish a strong airflow they also have low inhalation volumes. This severely impedes their ability to effectively clear the aerosol created and stored in a holding chamber such as that used by Exubera® (Nektar, San Carlos, CA).

Nebulizers, such as the jet nebulizers, produce a fine aerosol mist/droplets which carry the drug either as a suspension or dissolved in the aqueous medium. The jet nebulizers use compressed air to atomize the aqueous solution. The flow rate of the compressed air should be matched to the inhalation flow rate of the patient for optimum delivery of the drug. The patient can be administered the drug with repetitive non-forced inhalation over a prolonged period of time. The amount of drug delivered is influenced by a large number of factors such as viscosity, volume of drug fill, surface tension, inhalation flow, etc. The amount of drug delivered ranges from 3% to 6% for pediatric patients and 3% to 13% for adults. For pediatric delivery the nebulizers are normally coupled to a face mask. Since the nebulizer continues to produce the aerosol during the exhale cycle of the breath this leads to drug wastage, increased exposure of the drug to the patient's face and eyes and also to the care-giver. The disadvantages of nebulizers in general are their poor efficiency of delivery to the patient, a requirement for a compressor or compressed air and long delivery times, on the order of 5 to 15 minutes, etc.

Thus there is a need for a delivery mechanism for infants and young children, and also for respiratory compromised patients that overcomes the aforesaid and other disadvantages of the prior art, in a manner that delivers the drug efficiently, does not require inhalation co-ordination, operates under low inhalation volume, minimizes the exposure of the care giver to the drug, delivers the drug in a short time (preferably less than a minute), and is low cost and portable.

CA 2 811 947 A1 relates to an inhaler.

US 2011/000481 A1 relates to a nebulizer for infants and respiratory compromised patients.

The present invention provides an inhaler according to claim 1. More particularly, in accordance with the present invention there is provided a device, its use and method for aerosolized dosing of dry powder pharmaceutical preparations, or pharmaceutical agents dissolved or suspended in a liquid medium comprising a pharmaceutical aerosolization engine comprising a vibratory device. In one embodiment, the aerosolization engine is connected to a face mask and permits manual activation of the aerosolization engine by a caregiver, and presentation of aerosolized medication into the face mask. The face mask may be replaced with a nasal cannula or a mouth piece or other form of interface and the manual activation may be replaced with automated activation of the aerosolization engine through sensing of the patients' inhalation or tidal breathing maneuver, or through synchronization with hospital equipment operating to assist or substitute for the patient's breathing as in ventilators or in delivering oxygen or humidified air for example.

The disclosure also provides a method for automating the involuntary delivery of a pharmaceutical to the airway of a human or animal patient, utilizing the inhaler described immediately above. The method includes measuring the breathing pattern of the patient with the pressure sensor, temperature sensor, microphone, or other sensor with an output proportional to flow rate and determining the duration of a typical breath of the patient. After releasing a dose of the pharmaceutical into the aerosol chamber, and upon sensing the beginning of a single breath of the patient, the vibrating device is operated, which in connection with the aerosol chamber, produces a synthetic jet which expels the deaggregated pharmaceutical into the interface. The operation of the vibrating device is controlled to occur at the beginning of the patient's breath and for a specific duration which is calculated as a fraction of the patient's typical breath. As necessary, the steps of sensing the beginning of a breath and operating the vibrating device may be repeated over a series of breaths, or intermittently over a series of breaths, in order to ensure substantial delivery of the pharmaceutical to the airway of the patient.

The present invention has particular utility in connection with aerosolization and delivery of dry powdered pharmaceutical agents to an infant or small child or a respiratory-comprised patient and will be described in connection with such utility, although other utilities including continuous or semi-continuous or intermittent nebulization of dry powder pharmaceutical agents, pharmaceutical agents dissolved or suspended in a liquid medium, and delivery to infants and small children, respiratory-compromised patients, ventilated patients and unconscious patients is also contemplated.

Features and advantages of the present invention will be seen from the following detailed description, taken into conjunction with the accompanying drawings, wherein:
FIG. 1 is a drawing of a pharmaceutical delivery device according to one example described herein;
FIG. 2 is a drawing demonstrating one possible implementation of the device shown in FIG. 1;
FIGS. 3A and 3B are drawings of a pharmaceutical delivery device according to another alternative to the example shown in FIG. 1; and
FIG. 4 is a schematic demonstrating the operation of a pharmaceutical delivery device in accordance with the examples shown in FIGS. 1-3;
FIGS. 5, 6A and 6B are charts showing an example of the operation of a pharmaceutical delivery device in accordance with the present invention.
FIG. 7 is a perspective view of a hand-held pediatric nebulizer for use in explaining the invention;
FIG. 8 is a top plan view of the device of FIG. 7;
FIG. 9 is a bottom plan view showing details of the facemask portion of the device of FIG. 7;
FIG. 10 is a schematic diagram illustrating generation of nebulized powder medication in accordance with the present invention;
FIG. 11 is a perspective view illustrating a pharmaceutical package in accordance with a preferred embodiment of the invention; and
FIG. 12 is a flow diagram illustrating another embodiment of the invention.

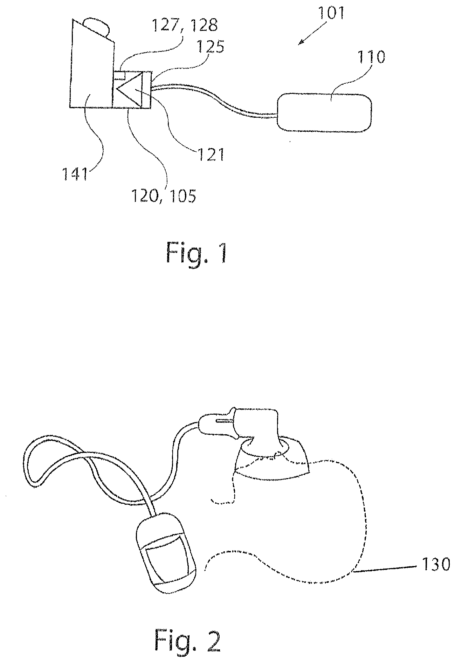

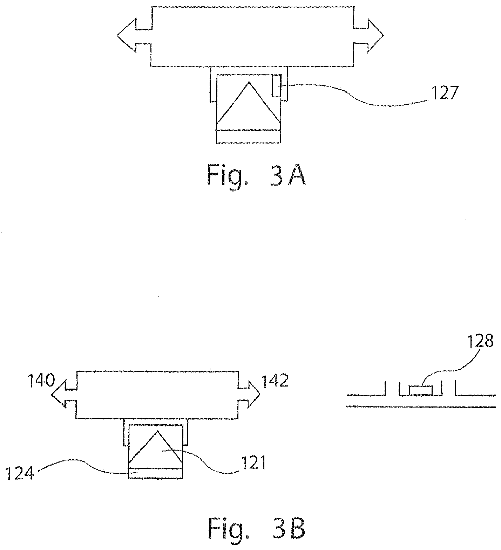

Referring to FIGS. 1-5, the present invention in one aspect provides a device and method for automatically delivering a pharmaceutical to the airway of a human or animal patient. The present invention enables the efficient dispensing of a pharmaceutical to a patient that is either unable to use or has substantial difficulty with prior art inhalers such as described elsewhere. Commonly-owned patent, U.S. Pat. No. 7,343,914 describes mouthpiece adapters suitable for use by children in connection with a typical dry-powder inhaler (DPI). However, other patients, such as infants, frail or pulmonary compromised adults, and most animals, are still unable to use or have difficulty using such inhalers.

The present invention therefore provides an inhaler that may be paired with a mask or other breathing apparatus and operated to ensure substantial inhalation of a dose of a pharmaceutical. The inhaler 101 includes an electronics housing 110 for containing the battery and other electronics associated with the control of the device. The inhaler also includes a delivery housing 120, which contains the vibrating device 125, aerosol chamber 121, and at least one dose 105 of a pharmaceutical. The housing is connected to an interface, such as a facemask 141 or nasal cannula 142, or other breathing apparatus known in the art. The device is arranged so that upon operation of the vibrating device, e.g., a piezoelectric device, the dose 105 is expelled from the aerosol chamber 121 into the interface for inhalation by the patient 130, via a synthetic jet (as described above and in our afore-mentioned applications). The inhaler also includes a pressure sensor 117, and/or a temperature sensor 118, (or other sensor) to measure the patient's breathing. The sensor(s) may be located within the housing, or alternatively, as part of the interface.

Because the present invention is intended to be used by a patient unable to use or use with difficulty a currently-available inhaler, it is necessary to devise a different operational scheme to ensure that the patient is able to inhale a substantial amount of the dose, even with tidal breathing.

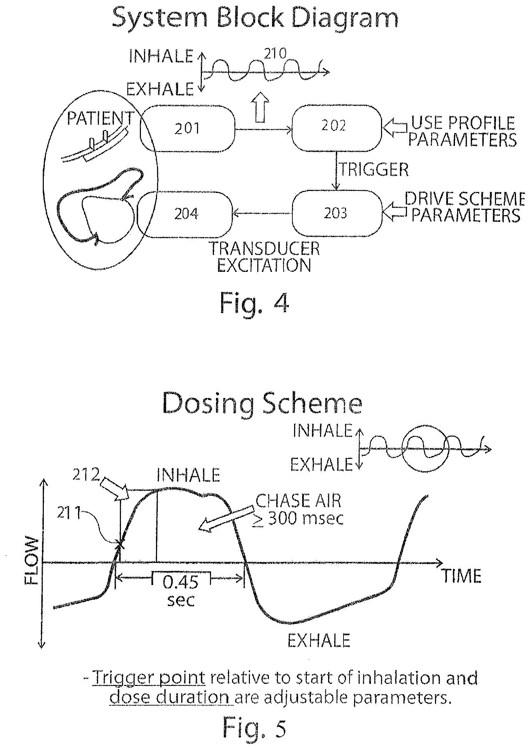

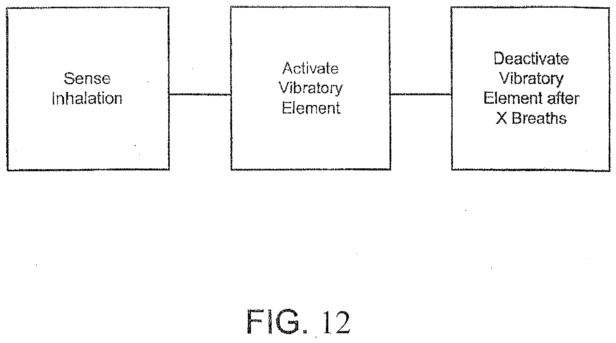

As shown in FIG. 4, at step 201 the pressure and/or temperature sensors are used to sense the breathing pattern 210 of the patient. This signal is processed at step 202 to determine the trigger point 211 and the dosing duration 212. (See FIG. 12). At step 203, these parameters are then sent to the electronics to control the dispensing of the dose and the operation of the vibrating device. Finally, at step 204, the dose is placed in the aerosol chamber and the vibrating device is operated according to the established parameters, which may include operating the device over a series of breaths in order to completely deliver the dose.

FIG. 5 shows the operational parameters in connection with a single breath of a patient. Because the patient's breathing could be labored or at a resting pattern, resulting in low inspiratory flow rates and volumes the dosing duration 212 should be much shorter that the dosing duration used in currently available inhalers. By way of example, the dosing duration may be calculated to be efficient at approximately 25% of the duration of the breath, or roughly one half of the duration of a typical inhaler. Shorter durations may be necessary.

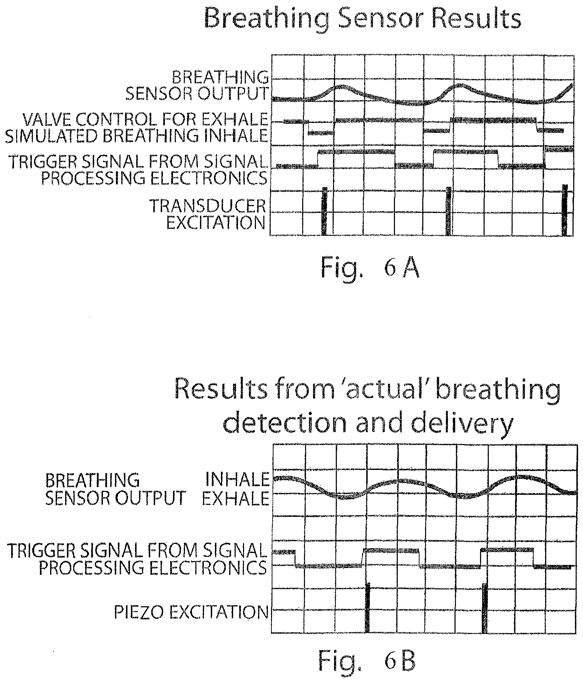

Further, as shown in FIGS. 6A and 6B, the inhaler may need to be operated over a series of breaths to fully dispense the pharmaceutical and ensure that the patient has inhaled a substantial amount of the dose. Alternatively, the inhaler may be operated intermittently over a series of breaths which may be timed to coincide with the patient's inhalation, or independent of the patient's inhalation. The number of breaths and the dosing duration may be determined according to the breathing pattern of the patient.

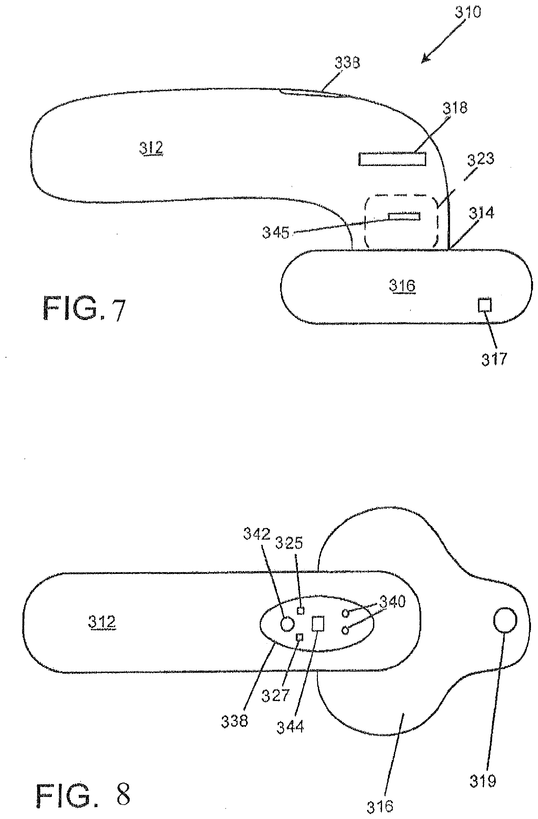

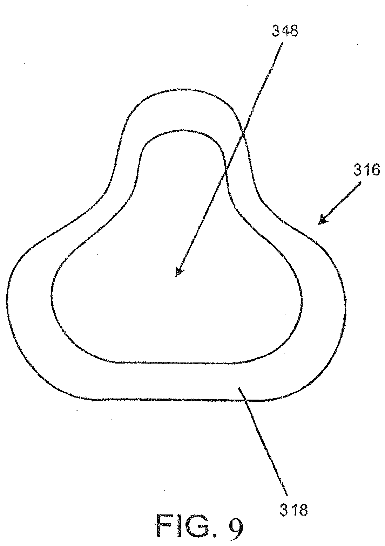

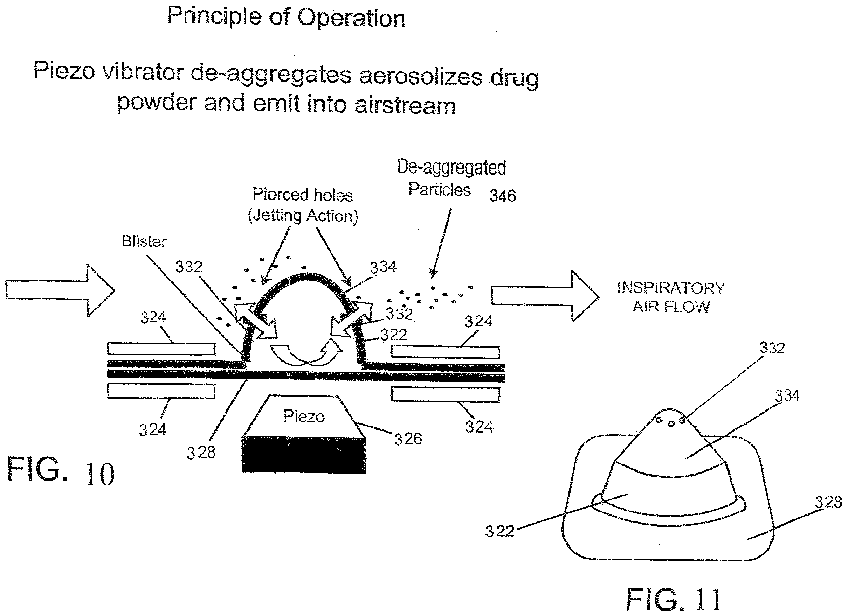

Turning now to FIGS. 7-12 of the drawings, there is illustrated a dry powder pediatric nebulizer for use in explaining the present invention. The nebulizer 310 comprises a housing or body 312 sized and shaped to fit comfortably within the hand of a human adult. Body 312 houses a dry powder aerosol engine, battery power and controls all as will be discussed below. Referring in particular to FIGS. 8 and 9, the hand held nebulizer 310 is connected at its outlet 314 to a facemask 316. Facemask 316 is sized and shaped to fit over the mouth and nose of a patient, and is formed of a resiliently deformable material such as silicon rubber. Facemask 316 may comprise a single wall construction or, if desired may comprise a soft partially air-filled cuff at its distal end 318, and optionally may include a one-way filter valve 319 to allow the patient's exhale breath to escape. Facemask 316 is friction fitted to the outlet end of nebulizer device 312 so that it may be removed for
cleaning and/or disposal and a fresh facemask placed thereon. Also, if desired, facemask 316 may come in different sizes, e.g. for adults, children and infants. The face mask may incorporate a pressure sensor 317 to measure the quality of fit and seal over the patient or the sensor may be incorporated into the inhaler housing. A good seal is preferred to ensure high efficiency of delivery of the drug to the patient and to protect the care-giver from exposure to the drug and the patient from exposure of the drug to the eyes.

Referring also to FIGS. 9-11 body 312 includes a movable panel 318 for permitting one or more molded bodies or blister packs 322 containing a powdered medication to be introduced into a chamber 323 (shown in phantom) defined within the interior of body 312. Blister pack 322 is guided by guides 324 to locate in contact with the top surface of an aerosolization engine in the form of a vibratory element 326. Alternatively, blister packs 322 may be a molded body that is reused over a number of dosings. The body in this case provides a way for introducing the drug into the chamber. Vibratory element 326 preferably comprises a piezo activator or piezo transducer or a mechanical vibrator, an electro-mechanical vibrator or a magnetostrictive element or other vibratory mechanism. Preferred are aerosolization engines and aerosolization chambers such as described in U.S. Patent Nos. 6,026,809, 6,142,146, 6,152,130, 7,318,434, 7,334,577, 7,343,914 and published U.S. Application Nos. 2005/0172962 and 2008/0202514.

Blister pack 322 preferably comprises a domed dry powder drug package made of cold formed or thermal formed film, and includes a conical, semi-spherical, elliptical, pyradidal or similar top part 334 and flat base 328 such as described in U.S. 7,080,644, assigned to the common assignee. Blister pack 322 has at least one drug ejection aperature 332 substantially opposite base 328 and serving primarily for injection of drug particles. Aperatures 332 may be pre-formed integrally with blister pack 322, or formed as puncture holes when the blister pack 322 is inserted into body 312.

Blister pack 322 carries a supply of a drug substance or substances which preferably are provided as a dry powder. A single component or several drug combinations may be used, or, the drug substance or substances combined with excipients, such as lactose or combinations thereof. Other additives such as pharmaceutically inactive ingredients, de-aggregation agents, etc., also may be added.

Body 312 carries a battery 325 for powering the vibratory element 326, as well as a microprocessor or electronic controller 327 for controlling operation of the vibratory element 336, sensor signal processing for inhalation and/or exhalation detection, etc. Body 312 also includes a control panel 338 including one or more activation buttons 340, 342, and a display 344. The display 344 may incorporate active dose feedbacks to indicate such things as device readiness, face mask seal integrity, activation of the aerosol engine during inhalation or tidal breathing and dosing complete, such as described in U.S. Published Application No. US-2005-0183725-A1. Body 312 also includes one or more side walled aperatures 345 which permit air to enter chamber (shown in phantom at 323) from the outside.

Operation of the nebulizer is as described below.

A caregiver places the facemask over the mouth and nose of the patient. Thereafter, the caregiver presses the start button 340 which activates the vibrating element 326 for a predetermined time, e.g. 1-2 seconds. The vibrating element engages with the base of blister pack 322 whereupon powdered medication is deaggregated and ejected out of blister pack 334 into chamber 323 as a cloud or powder plume 346 where it is then inhaled by the patient.

The present invention has several advantages over the prior art. For one, the ability to aerosolize dry powders and deliver same in a nebulizer permits much higher dose concentrations than are possible with liquid carried drugs. Thus, administration time for a dose may be significantly reduced over those of a liquid nebulizer. Also, many drugs are insoluble in water and can't be delivered using conventional nebulizers, or are soluble only in organic solvents which create other problems.

Another feature and advantage of the present invention is that the generation of powder plume is independent of inhalation rate and inhalation timing. Thus, the nebulizer of the present invention is particularly useful in the case of infants and small children, respiratory compromised patients, and unconscious patients. The above described invention provides controlled, reproducible and recordable pulmonary doses from pre-measured blister packs. Alternatively, a plurality of blister packs may be mounted in the body 312 as a cartridge, and advanced, as necessary. Alternatively the dose amount may be controlled by the number and duration of the delivery 'pulses', or aerosol activation cycles.

The invention is susceptible to modification. For example, facemask 316 may be removed, or the nebulizer mounted directly to a pre-existing ventilator/nebulizing system where it may be run intermittedly. The nebulizer also may be triggered to turn on and off by sensing tidal breathing of a patient as illustrated in Fig. 4 and 12, and operate over one or several breaths. Referring again to Figs. 5, 6A and 6B, the inhalation and/or exhalation cycle is sensed and the aerosol generator is turned on for a short duration followed by an amount of chase air to carry or follow the particles into the patient. A sufficient quantity of chase air is necessary to ensure lung deposition when inhalation volumes are low and inhalation cycles are short. Any sensor or combination of sensors that can be used to measure or identify the difference in properties between an inhalation and exhalation manuever can be used to synchronize and turn the aerosol generator on and off. Example of sensors that may be used to detect the patients inhalation/exhalation are flow sensors, pressure sensors, temperature sensors that measure the temperature difference between the inhaled and exhaled breath, carbon dioxide or nitric oxide or other gas sensors that measure the gas component level difference between inhaled and exhaled breath, and also physical measurement systems such as chest straps to measure the expansion and contraction of the chest cavity, etc., can be employed for this purpose. Still other changes are possible. For example, active visual, audible or tactile feedback to the patient or caregiver indicating the status of the device and of dosing may be provided including, for example, visual or audible devices as taught in U.S. Patent 7,343,914.
Also, if desired, electronic communication may be provided for connecting the device to equipment connected to the patient for controlling or synchronizing the vibratory clement, Also, if desired, the dose or amount delivered to a patient may be determined by the counting and controlling number of timed or pulsed activations of the vibratory element. Also animal or cartoon images may be printed on the inside surface 348 of the facemask 316, to make the instrument more friendly to a child patient, or the device feedback systems, e.g. lights and sounds and vibrations may be used for this purpose.

A feature and advantage of the present invention is that it provides a mechanism to allow delivery of inhalation therapy to patients not currently served by current commercial inhalers.

Also, while the invention has been described in particular for use with drugs for treating asthma and COPD, the invention also advantageously may be used for delivery of other drugs including, but not limited to, anti-virals to treat viruses including but not limited to RSV, and anti-biotics, anti-fungals and anti-infectives for treating lung infections and other diseases, or drugs for treating lung cancer.

Still other changes are possible. For example, it is possible to control the amount of drug delivered to the nasal passages as opposed to just the lower respiratory track by controlling particle size. Still other changes are possible.

## Claims

1. An inhaler (101) for delivering a pharmaceutical to the airway of a patient, comprising:
a first housing (120) containing:
at least one dose of a pharmaceutical in dry powder form;
a pressure sensor (117) located within the first housing (120) to measure the patient's breathing;
a vibrating device (125) for aerosolizing the dry powder pharmaceutical;
an aerosol chamber (121); and
an interface for delivering the pharmaceutical to the patient,
wherein the inhaler (101) is **characterized by** a second housing (110) containing electronics for monitoring the pressure sensor (117) and for controlling the operation of the vibrating device (125),
wherein the inhaler is arranged so that the pressure sensor (117) measures the patient's breathing and parameters are sent to the electronics to control the dispensing of the dose and operation of the vibrating device (125),
wherein the vibrating device (125) is arranged to be operated intermittently over a series of breaths to dispense the dose and operation of the vibrating device coincides with the patient's inhalations, and
wherein the inhaler is arranged so that upon operation of the vibrating device (125) the dose is expelled from the aerosol chamber (121) via a synthetic jet into the interface for inhalation by the patient.

2. The inhaler (101) of claim 1, further comprising a temperature sensor (118) or other sensor located within the first housing (120) to measure the patient's breathing, wherein the inhaler is arranged so that the pressure sensor (117) and the temperature sensor (118) or other sensor measures the patient's breathing and parameters are sent to the electronics to control the dispensing of the dose and operation of the vibrating device (125).

## Patentansprüche

1. Inhalator (101) zum Verabreichen eines Arzneimittels in den Atemweg eines Patienten, umfassend:
ein erstes Gehäuse (120), das Folgendes enthält:
mindestens eine Dosis eines Arzneimittels in Trockenpulverform;
einen Drucksensor (117), der im ersten Gehäuse (120) positioniert ist, um die Atmung des Patienten zu messen;
eine Vibrationsvorrichtung (125) zum Aerosolieren des Trockenpulverarzneimittels;
eine Aerosolkammer (121); und
eine Schnittstelle zum Verabreichen des Arzneimittels an den Patienten,
wobei der Inhalator (101) durch ein zweites Gehäuse (110) gekennzeichnet ist, das Elektronik zum Überwachen des Drucksensors (117) und zum Steuern des Betriebs der Vibrationsvorrichtung (125) enthält,
wobei der Inhalator so angeordnet ist, dass der Drucksensor (117) die Atmung des Patienten misst und Parameter an die Elektronik gesendet werden, um die Abgabe der Dosis und den Betrieb der Vibrationsvorrichtung (125) zu steuern,
wobei die Vibrationsvorrichtung (125) angeordnet ist, um intermittierend über eine Reihe von Atemzügen hinweg betrieben zu werden, um die Dosis abzugeben, und der Betrieb der Vibrationsvorrichtung mit den Inhalationen des Patienten zusammenfällt und
wobei der Inhalator so angeordnet ist, dass bei Betrieb der Vibrationsvorrichtung (125) die Dosis über einen synthetischen Strahl aus der Aerosolkammer (121) für eine Inhalation durch den Patienten in die Schnittstelle ausgestoßen wird.

2. Inhalator (101) nach Anspruch 1, ferner umfassend einen Temperatursensor (118) oder einen anderen Sensor, der im erste Gehäuse (120) positioniert ist, um die Atmung des Patienten zu messen, wobei der Inhalator so angeordnet ist, dass der Drucksensor (117) und der Temperatursensor (118) oder der andere Sensor die Atmung des Patienten misst und Parameter an die Elektronik gesendet werden, um die Abgabe der Dosis und den Betrieb der Vibrationsvorrichtung (125) zu steuern.

## Revendications

1. Inhalateur (101) pour administrer un médicament aux voies aériennes d'un patient, comprenant :
un premier logement (120) contenant :
au moins une dose d'un médicament sous forme de poudre sèche ;
un capteur de pression (117) situé à l'intérieur du premier logement (120) pour mesurer la respiration du patient ;
un dispositif vibrant (125) pour aérosoliser le médicament en poudre sèche ;
une chambre aérosol (121) ; et
une interface pour administrer le médicament au patient,
l'inhalateur (101) étant **caractérisé par** un deuxième logement (110) contenant des composants électroniques pour surveiller le capteur de pression (117) et pour commander le fonctionnement du dispositif vibrant (125),
l'inhalateur étant agencé de telle sorte que le capteur de pression (117) mesure la respiration du patient, les paramètres étant envoyés aux composants électroniques pour commander l'administration de la dose et le fonctionnement du dispositif vibrant (125),
le dispositif vibrant (125) étant agencé pour être actionné de manière intermittente au cours d'une série de respirations pour administrer la dose, le fonctionnement du dispositif vibrant coïncidant avec les inhalations du patient, et
l'inhalateur étant agencé de telle sorte que, lors du fonctionnement du dispositif vibrant (125), la dose est expulsée de la chambre aérosol (121) dans l'interface via un jet synthétique pour être inhalée par le patient.

2. Inhalateur (101) selon la revendication 1, comprenant en outre un capteur de température (118) ou autre capteur situé à l'intérieur du premier logement (120) pour mesurer la respiration du patient, l'inhalateur étant agencé de telle sorte que le capteur de pression (117) et le capteur de température (118) ou autre capteur mesurent la respiration du patient, les paramètres étant envoyés aux composants électroniques pour commander l'administration de la dose et le fonctionnement du dispositif vibrant (125).
